# EUROPEAN PATENT APPLICATION

(11) **EP 1 391 731 A1**
(43) Date of publication of application: **25.02.2004**
(21) Application number: 03017786.9
(22) Date of filing: 04.08.2003
(51) Int. Cl.: G01N 33/58, G01N 33/533, C12Q 1/68, G01N 1/30, G01N 21/64

(54) **Functional fluorescence reagent comprising semiconductor nanoparticles**

(30) Priority: 21.08.2002 JP 2002240848
(71) Applicant: Hitachi Software Engineering Co., Ltd., Yokohama-shi, Kanagawa 230-0045 (JP)
(72) Inventor: Sato, Keiichi, Hitachi Software Engineering Co. Ld, Tokyo 140-0002 (JP); Kuwabata, Susumu, c/o Engineering Osaka University, Suita-shi, Osaka 565-0871 (JP)
(74) Representative: Liesegang, Roland, Dr.-Ing.

(57) **Abstract**

This invention provides semiconductor nanoparticles having excellent fluorescence properties and capable of fusing with biopolymers. The semiconductor nanoparticles are encapsulated in a phospholipid bilayer membrane or phospholipid multi-layer membrane to impart functionality thereto. Thus, functional semiconductor nanoparticles can be produced while fluorescence properties of semiconductor nanoparticles are maintained.

## Description

### Technical Field

The present invention relates to a functional fluorescent substance comprising semiconductor nanoparticles which can, for example, modify or stain substances associated with organisms. The present invention also relates to bioassay or other applications which utilize the fluorescence properties of this fluorescent substance.

### Background Art

Semiconductor nanoparticles whose particle sizes are 10 nm or less are located in the transition region between bulk semiconductor crystals and molecules. Their physicochemical properties are therefore different from both bulk semiconductor crystals and molecules. In this region, due to the quantum-size effect, the energy gap of semiconductor nanoparticles increases as their particle sizes decrease. In addition, the degeneration of the energy band that is observed in bulk semiconductors is removed and the orbits are dispersed. As a result, the lower-end of the conduction band is shifted to the negative side and the upper-end of the valence band is shifted to the positive side.

Semiconductor nanoparticles of CdX (X being S, Se or Te) can be easily prepared by dissolving equimolar amounts of precursors of Cd and X. This is also true for the manufacturing of, for example, ZnS, ZnSe, HgS, HgSe, PbS, or PbSe.

Semiconductor nanoparticles have drawn attention since they emit strong fluorescences whose full widths at half maximum are narrow. Thus, various fluorescent colors can be created, and their future applications can be nearly infinite. However, the semiconductor nanoparticles obtained only by mixing the precursors with each other as described above have a wide distribution of particle sizes and therefore cannot provide the full advantage of the properties of semiconductor nanoparticles. Attempts have been made to attain a monodisperse distribution by using chemical techniques to precisely separate and extract only the semiconductor nanoparticles of a specific particle size from semiconductor nanoparticles having a wide distribution of particle sizes immediately after preparation. The attempts to attain a monodispersed distribution of particle sizes that have been reported so far include: separation by electrophoresis that utilizes variation in the surface charge of nanoparticles depending on their particle sizes; exclusion chromatography that utilizes differences in retention time due to different particle sizes; and size-selective precipitation that utilizes differences in dispersibility in an organic solvent due to differences in particle sizes.

A method was described above wherein the nanoparticles, which were prepared by mixing the precursors with each other, were separated depending on their particle sizes. Also reported is size-selective photoetching that attains a monodispersed distribution of particle sizes by utilizing the oxidative dissolution of a metal chalcogenide semiconductor in the presence of dissolved oxygen when irradiated with light.

There is also a method wherein a monodispersed distribution of particle sizes is attained through regulation at the phase of mixing the precursors with each other. A representative example thereof is the reversed micelle method. In the reversed micelle method, amphiphilic molecules such as diisooctyl sodium sulfosuccinate are mixed with water in an organic solvent such as heptane to form a reversed micelle therein, and precursors are allowed to react with each other only in an aqueous phase in the reversed micelle. The size of the reversed micelle is determined according to the quantitative ratio of the amphiphilic molecules to water, and its size can be relatively homogenously regulated. The sizes of prepared semiconductor nanoparticles depend on the size of the reversed micelle. Thus, semiconductor nanoparticles with relatively homogenous particle sizes can be prepared.

The thus prepared semiconductor nanoparticles exhibit a relatively narrow distribution of particle sizes. However, the fluorescence properties of the thus prepared semiconductor nanoparticles show smooth fluorescence emission spectra without any significant peaks. Further, the fluorescence emission spectra exhibit a peak at a wavelength that differs from the theoretical value of the fluorescence, which should be emitted by the semiconductor nanoparticles. Specifically, in addition to the band gap fluorescence emitted inside the semiconductor nanoparticles, the semiconductor nanoparticles emit completely different fluorescences, which are supposed to be emitted at the energy level in the forbidden band in the semiconductor particles. The energy level that emits this fluorescence is considered to exist mainly at the surface site of the semiconductor nanoparticles. Under normal circumstances, changes in fluorescence properties due to the particle size regulation of the semiconductor nanoparticles appear in the band gap fluorescence. This inhibits the properties of semiconductor nanoparticles having a narrow distribution of particle sizes, and thus, it has been a problem that should be solved.

As a representative solution for this problem, a method has been attempted wherein a semiconductor material, which is a nucleus, is coated with a semiconductor material, inorganic material, or organic material having a band gap wider than that of the aforementioned semiconductor material to attain a multi-layer structure, and fluorescences thereof are inhibited. Examples of particularly representative methods of coating the semiconductor nanoparticles with inorganic materials include: coating of CdSe nanoparticles with CdS (J. Phys. Chem. 100: 8927 (1996)); coating of CdS nanoparticles with ZnS (J. Phys. Chem. 92: 6320 (1988)); and coating of CdSe nanoparticles with ZnS (J. Am. Chem. Soc. 112: 1327 (1990)). Regarding the CdSe nanoparticles coated with ZnS (J. Am. Chem. Soc. 112: 1327 (1990)), with the utilization of the Ostwald ripening, a production method that is carried out in the coordinated solvent is adopted, thereby successfully obtaining semiconductor nanoparticles having sufficient fluorescence properties (J. Phys. Chem. B. 101: 9463 (1997)). The aforementioned multi-layered semiconductor nanoparticles inhibit the defective site on the surface of the semiconductor nanoparticles and attain the original fluorescence properties of the semiconductor nanoparticles by coating them with a material having a band gap larger than that of the semiconductor nanoparticles and having no band gap in the forbidden band of the semiconductor nanoparticles.

Surface treatment of semiconductor nanoparticles by methods as mentioned above can inhibit defective sites to some extent, and this realizes the production of semiconductor nanoparticles having sufficient fluorescence properties.

The surfaces of the thus obtained semiconductor nanoparticles can be easily modified with a thiol compound or the like. Accordingly, semiconductor nanoparticles having a specific functional group exposed thereon can be produced. When semiconductor nanoparticles are modified with a thiol compound, however, their fluorescence properties are significantly deteriorated in many cases. Research has also been in progress regarding the stability of thiol-modified semiconductor nanoparticles. For example, Peng et al. discussed the stability of mercaptopropionic acid-modified CdSe nanoparticles in J. Am. Chem. Soc. 123: 8844 (2001), although they did not provide favorable results therein.

Meanwhile, research on vesicles using phospholipid bilayer membranes has drawn much attention in the field of drug delivery systems (DDS), the main object of which is the local administration of a drug. This research has made rapid advances. This makes use of properties such as the ability of immobilization of a protein and an antibody on a phospholipid bilayer membrane to impart functionality such as recognition of organism tissues, and the ability of the fusion of a vesicle bound to a cell and the cell to incorporate a substance existing in the vesicle into the cell. For example, when treating cancer, if a drug can be directly administered to a cancer cell exclusively, side effects caused by an anticancer agent can be significantly decreased. Accordingly, the effects attained by its utilization are very significant.

Several attempts have been made to produce semiconductor nanoparticles using a vesicle. A representative example thereof is the production of semiconductor nanoparticles in a vesicle (Korgel et al., Langmuir. 16: 3588 (2000)). In this report, however, there is only one semiconductor nanoparticle in a vesicle. In addition, it was aimed at the isolation of a semiconductor nanoparticle and was not intended to impart functionality such as the existence of fluorescence properties. There is no other report concerning the impartation of such functionality.

Regarding the flowmetry-based bioassay using polystyrene beads, a product has been developed and is already commercially available from Luminex. This product measures the amounts of a sample bound to a biopolymer and an antibody immobilized on the surface of polystyrene beads by imparting identification in the polystyrene beads based on different quantitative ratios of fluorescent dyes, allowing them to react with a fluorescence-modified sample on the surface of the polystyrene beads having specific biopolymer and antibody immobilized thereon, and simultaneously reading the beads and their surfaces one by one by flowmetry after the reaction.

As described above, the surfaces of the semiconductor nanoparticles should be modified in order to impart functionality thereto. Direct surface modification, however, was not preferable from the viewpoint of maintaining the fluorescence properties of the nanoparticles.

### Summary of the Invention

Accordingly, an object of the present invention is to develop semiconductor nanoparticles with excellent fluorescence properties and the ability to fuse with biopolymers.

The present inventors have conducted concentrated studies. As a result, they have found that the surface conditions of the semiconductor nanoparticles can be maintained by encapsulation of semiconductor nanoparticles in a capsule constituted by a vesicle comprising a phospholipid bilayer membrane, etc. as a means of allowing semiconductor nanoparticles to have fluorescence properties and functionality as fluorescent substances. Thus, semiconductor nanoparticles can be used as functional fluorescent substances having luminescence properties. This has led to the completion of the present invention.

More specifically, the first aspect of the present invention relates to a functional fluorescent substance that comprises semiconductor nanoparticles in a vesicle and has fluorescence properties.

A "vesicle" is a kind of artificial membrane, which is also referred to as a "liposome." This refers to a closed follicle autonomously comprising a bilayer membrane, which is formed when a phospholipid is suspended in a buffer and allowed to stand at a temperature equal to or higher than its phase transition temperature. When vesicles are produced with the application of mechanical oscillation, multiple concentric follicles having heterogeneous diameters of 0.1 to 1 µm are produced. This is referred to as a "multi-layer vesicle (multi-layer liposome)" or "multilamellar liposome." Further, ultrasonication can realize the production of vesicles (liposomes) comprising a lipid bilayer membrane of a relatively uniform size (diameter: 20 to 50 nm). This is referred to as a lipid bilayer membrane vesicle (single compartment liposome) or unilamellar liposome. A larger lipid bilayer membrane vesicle can be prepared by dissolving a phospholipid in an organic solvent (e.g., ether), adding a buffer thereto, and eliminating the organic solvent using a tap aspirator. In this case, the diameter is approximately 200 to 500 nm. Cholesterol, glycolipid, acylglycerol, or the like can be added to the vesicle, a membrane protein can be further incorporated in the vesicle, and these can be used for studying structures or functions of biomembranes. In addition, the movement, phase transition, phase separation, and the like of lipid molecules have been studied. Since a vesicle is a closed follicle containing water therein, it can sustain a water-soluble ion, low molecular weight substance, protein, or the like inside itself. Thus, it is also used as a carrier, which delivers a drug to a specific lesion (DDS) by encapsulating an anticancer agent such as adriamycin in a liposome. When this type of vesicle is allowed to fuse with a cell membrane, it is useful to introduce, for example, a high molecular weight substance or plasmid, which cannot permeate the cell membrane, into a cell.

The term "semiconductor nanoparticles" used in the present invention refers to compound semiconductor particles having sizes on the nanometer order. The compound semiconductor particles are represented by a general formula MX, wherein M is a metal atom and selected from Zn, Cd, Hg, In, Ga, Ti, W, Pb, and the like. X is selected from O, S, Se, Te, P, As, N, and the like. Specific examples thereof include ZnO, ZnS, ZnSe, ZnTe, CdO, CdS, CdSe, CdTe, HgS, HgSe, HgTe, InP, InAs, GaN, GaP, GaAs, TiO₂, WO₃, PbS, and PbSe. Those having hydroxyl groups on the surfaces of semiconductor nanoparticles can be preferably used for stabilization.

The semiconductor nanoparticles of the present invention have significant fluorescence properties. In particular, the fluorescence properties are strongly exhibited when the semiconductor nanoparticles have a monodispersed distribution of particle sizes. More specifically, the particle sizes of the semiconductor nanoparticles are preferably monodispersed, so that deviations are less than 10% rms in diameter. Methods for monodispersing the particle sizes of the semiconductor nanoparticles are not limited, and examples thereof include conventional methods such as separation by electrophoresis, exclusion chromatography, size-selective precipitation, size-selective photoetching, and the reverse micelle method.

The fluorescence emitted by the semiconductor nanoparticles of the present invention has a sharp peak of fluorescence intensity. The semiconductor nanoparticles can also emit fluorescence in a narrow spectrum range of 60 nm or less in terms of the full width at half maximum (FWHM). It is preferably 40 nm or less, and more preferably 30 nm or less in terms of the full width at half maximum (FWHM).

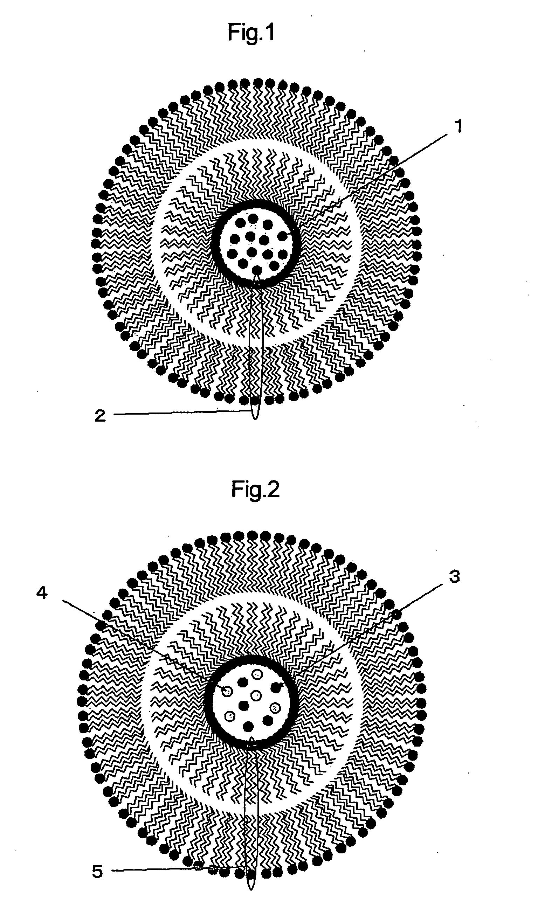

In the present invention, the number of semiconductor nanoparticles contained in a vesicle is not limited, and a vesicle can contain from one to tens of thousands of particles or more depending on its size. Fig. 1 is a pattern diagram showing a functional fluorescence reagent according to the present invention. In Fig. 1, semiconductor nanoparticles 1 are encapsulated in a vesicle formed by a phospholipid bilayer membrane 2. Particularly, one vesicle can contain two or more semiconductor nanoparticles. When the two or more semiconductor nanoparticles have different fluorescent wavelengths, the use thereof as a fluorescent labeling substance for cytometry or the like is very effective, as described below.

As described above, vesicles are classified into two types. One type thereof comprises a phospholipid bilayer membrane, and the other is those having a phospholipid multi-layer membrane. In the present invention, however, the vesicle is not limited to either type.

Phospholipid molecules constituting vesicles have a high affinity with biopolymers. Thus, the functional fluorescent substance according to the present invention includes vesicles comprising substances associated with organisms immobilized on their surfaces. Substances associated with organisms to be immobilized are not particularly limited, and preferable examples thereof include DNA, proteins, antibodies, and antigens.

The present invention relates to a fluorescence reagent that utilizes fluorescence properties of the semiconductor nanoparticles. This fluorescence reagent can be used as a testing reagent in the fields of biotechnology and medicine, and can also be used as a light emitting element utilizing luminescence at various wavelengths. Further, this fluorescence reagent can be applied to fields where conventional fluorescence reagents are used.

The second aspect of the present invention relates to a method for staining cells and organism tissues wherein the functional fluorescent substance is used as a stain for cells and organism tissues. For example, this can be applied to a fluorescence microscope wherein an excitation light such as an ultraviolet one is applied to fluorescent substances in cells or organism tissues to observe the fluorescence emitted, and to a contrast medium for organism tissues.

The third aspect of the present invention relates to an immunofluorescence method. This method includes both a method wherein an antibody, which is labeled with the functional fluorescent substance, is used to detect an antigen and a method wherein an antigen, which is labeled with the functional fluorescent substance, is used to detect an antibody.

The fourth aspect of the present invention relates to a bioassay method wherein the functional fluorescent substance is used for labeling in flow cytometry. The flow cytometry utilizing fluorescence assays the number of cells, individuals, and other biological particles that are suspended in a liquid and their physical, chemical, and biological properties. In this technique, samples are made to flow sequentially to the assay site, and based on the fluorescence, one particle can be subjected to sequential multichannel analysis. For example, DNA hybridization, the shape and the size of a cell, and expression of molecules by several types of monoclonal antibodies labeled with different fluorescent dyes can be analyzed. Further, this technique is extensively used in, for example, the separation of a specific cell group in cultured cells, lymphatic organs, or vascular flow, naturally-occurring water particles including planktons, and a specific chromosome among others.

In the present invention, an example of a particularly preferable bioassay method is one wherein the functional fluorescent substance, which is imparted with identification determined by the different quantitative ratio of fluorescent dyes, is allowed to react with a substance associated with organisms to detect the functional fluorescent substance by flow cytometry after the reaction.

The fifth aspect of the present invention relates to a bioassay device in which the functional fluorescent substance is used for labeling in flow cytometry.

### Brief Description of the Drawings

Fig. 1 is a pattern diagram showing the functional fluorescence reagent according to the present invention.
Fig. 2 is a pattern diagram showing a case where semiconductor nanoparticles having different fluorescence wavelengths are encapsulated in a single vesicle.

### Examples

### [Preparation of semiconductor nanoparticles]

Semiconductor nanoparticles were prepared in the following manner. At the outset, 1000 ml of aqueous solution comprising sodium hexametaphosphate (0.1 mmol) and cadmium perchlorate (0.2 mmol) was prepared and adjusted at a pH level of 10.3. Thereafter, the solution was subjected to bubbling using nitrogen gas, and hydrogen sulfide gas (0.2 mmol) was poured into the solution while vigorously stirring. Thereafter, stirring was carried out for a while. At this time, the color of the solution changed from optically transparent colorless to optically transparent yellow.

The semiconductor nanoparticles which have been already stabilized with hexametaphosphoric acid are already present in the solution. The semiconductor nanoparticles have a wide distribution of particle sizes, and the standard deviations reach up to 15% or more of the average particle sizes. The fluorescence intensity of the semiconductor nanoparticles in this state is very weak as a whole.

### [Monodispersion of semiconductor nanoparticles]

Size-selective photoetching is described. Due to the quantum-size effect, the physicochemical properties of semiconductor nanoparticles depend on their particle sizes. Accordingly, the physical properties of these semiconductor nanoparticles in this state are averaged out and their traits cannot be fully manifested. Thus, there is a need to use chemical techniques to precisely separate and extract only the semiconductor nanoparticles of a specific particle size from semiconductor nanoparticles having a wide distribution of particle sizes immediately after preparation in order to attain monodispersed distributions. One example of the method according to the above is size-selective photoetching. Size-selective photoetching takes advantage of the fact that the energy gap of a semiconductor nanoparticle increases due to the quantum-size effect as the particle size thereof decreases and that a metal chalcogenide semiconductor is oxidatively dissolved in the presence of dissolved oxygen when irradiated with light. In this method, the semiconductor nanoparticles having a wide distribution of particle sizes are irradiated with monochromatic light of a wavelength shorter than the wavelength of the semiconductor nanoparticle's absorption edge. This causes only the semiconductor nanoparticles of larger particle sizes to be selectively photoexcited and dissolved, thus sorting the semiconductor nanoparticles into smaller particle sizes.

At the outset, the thus obtained solution of semiconductor nanoparticles having a wide distribution of particle sizes, which was stabilized by hexametaphosphoric acid, is subjected to bubbling with nitrogen gas, followed by further bubbling with oxygen for 10 minutes. Thereafter, methyl viologen is added to the solution at 50 µmol/l and a laser was applied while stirring. The application of monochromatic light in the present invention is carried out for the purpose of photodissolution of the semiconductor nanoparticles. The wavelength of the monochromatic light was determined to be 450 nm. Variation of the wavelength of this monochromatic light can regulate the fluorescence wavelength at the peak in the fluorescence emission spectrum of the semiconductor nanoparticles.

When the semiconductor nanoparticles obtained by this method are irradiated with light having a wavelength of 476.5 nm, the average particle size is 3.2 nm and the standard deviation is 0.19 nm. Specifically, the standard deviation exhibits a very narrow distribution of particle sizes, i.e., approximately 6% of the average particle sizes. Thus, a solution of semiconductor nanoparticles with particle sizes very close to the monodispersed state can be obtained.

### [Stabilization of semiconductor nanoparticles]

In order to further purify the thus obtained monodispersed semiconductor nanoparticles that were stabilized with hexametaphosphoric acid, 300 µl of mercaptopropionic acid (MPA) was added, and the mixture was stirred for several hours to perform surface modification. Further, ultrafiltration was performed to remove methyl viologen, hexametaphosphoric acid, unreacted thiol compound, ions dissolved at the time of photoetching, or the like in the aqueous solution. Thus, a pure solution of semiconductor nanoparticles that were stabilized with a thiol compound was obtained.

### [Encapsulation of semiconductor nanoparticles in a vesicle]

The thus obtained semiconductor nanoparticles have fluorescence properties. There is a method for imparting functionality to nanoparticles wherein the surfaces of the semiconductor nanoparticles having fluorescence properties are directly modified. According to the reason presented above, it is undesirable to directly modify the surfaces of semiconductor nanoparticles to impart functionality. In the present invention, accordingly, semiconductor nanoparticles are encapsulated in a vesicle, and functionality is imparted to the surface of the vesicle, thereby imparting functionality to the fluorescent semiconductor nanoparticles. An example of a method for encapsulating semiconductor nanoparticles in a vesicle is hereafter provided.

Distearoyl phosphatidylcholine (DSPC) (0.036 g, 0.045 mmol) was placed in a stoppered test tube. About 2 ml of chloroform was added thereto to completely dissolve DSPC, and the chloroform was removed using a rotary evaporator. A thin film was then formed on an inner wall of the test tube. Thereafter, vacuum drying was carried out for several hours in order to completely remove the chloroform. Further, 4.5 ml of buffer comprising 20 mM Tris-HCl (pH 7.5) and a solution of semiconductor nanoparticles of a concentration at which the absorbance is 0.1 was applied to the thin film, the thin film was heated to 70°C to peel it off the inner wall of the test tube, and ultrasound was applied to the thin film using a 25 W probe-type sonicator in a hot water bath at approximately 70°C for 5 minutes. The resulting dispersion liquid was subjected to gel filtration using a gel filtration column (Sephadex G 50, 2.0 g) using a buffer comprising 20 mM Tris-HCl (pH 7.5) and 100 mM NaCl as an eluent. Thus, semiconductor nanoparticles encapsulated in a vesicle were isolated.

Semiconductor nanoparticles encapsulated in a vesicle were obtained in the above manner. The semiconductor nanoparticles can comprise biopolymers such as DNA, a protein, a lipid, or an antibody immobilized on their surfaces. Utilization of the semiconductor nanoparticles having the biopolymers immobilized thereon allows the exclusive staining of specific cells. When a vesicle capable of cell fusion is applied, cells can be directly stained. As mentioned above, semiconductor nanoparticles are significantly durable compared with general dyes. Accordingly, application thereof to real-time bioimaging or real-time medical treatment and diagnosis, which would become widely used in the future, can be expected.

### [Encapsulation of semiconductor nanoparticles having different fluorescence wavelengths]

In the present invention, semiconductor nanoparticles having different fluorescence wavelengths can be mixed and encapsulated in a vesicle, and identification can be imparted to the vesicle. Fig. 2 is a pattern diagram showing a case where semiconductor nanoparticles having different fluorescence wavelengths are encapsulated in a single vesicle. In Fig. 2, when the identification impartation is carried out by nanoparticles having two types of fluorescence wavelengths, a first semiconductor nanoparticle 3 and a second semiconductor nanoparticle 4 are encapsulated in a vesicle formed by a phospholipid bilayer membrane 5. This can impart a function equivalent to that of polystyrene beads in the flowmetry-based bioassay using the polystyrene beads, and the identification is determined based on the quantitative ratio between the first semiconductor nanoparticle 3 and the second semiconductor nanoparticle 4 having different fluorescence wavelengths. In this case, any number of types of different semiconductor nanoparticle may be employed.

A monolayer membrane vesicle was used in the examples. The same applies to a multi-layer membrane vesicle.

### Effect of the Invention

The present invention can provide an extensively functional fluorescent substance by combining highly durable semiconductor nanoparticles as fluorescent substances and an amphiphilic phospholipid bilayer membrane or phospholipid multi-layer membrane capable of binding and recognition. Since direct surface modification of semiconductor nanoparticles is not required, semiconductor nanoparticles can be imparted with functionality while their fluorescence properties are maintained.

The fluorescence reagent utilizing fluorescence properties of the semiconductor nanoparticles according to the present invention can be used as a testing reagent in the fields of biotechnology and medicine and as a light emitting element utilizing luminescence at various wavelengths. In addition, the fluorescence reagent according to the present invention can be applied in the fields where conventional fluorescence reagents are used.

## Claims

1. A functional fluorescent substance having fluorescence properties, wherein semiconductor nanoparticles are encapsulated in a vesicle.

2. The functional fluorescent substance according to claim 1, wherein two or more semiconductor nanoparticles are encapsulated in a vesicle.

3. The functional fluorescent substance according to claim 2, wherein the two or more semiconductor nanoparticles have different fluorescence wavelengths.

4. The functional fluorescent substance according to any one of claims 1 to 3, wherein the vesicle comprises a phospholipid bilayer membrane.

5. The functional fluorescent substance according to any one of claims 1 to 4, wherein the vesicle comprises a phospholipid multi-layer membrane.

6. The functional fluorescent substance according to any one of claims 1 to 5, wherein a substance associated with organisms is immobilized on the surface of the vesicle.

7. The functional fluorescent substance according to claim 6, wherein the substance associated with organisms is any of DNA, a protein, an antibody, or an antigen.

8. The functional fluorescent substance according to any of claims 1 to 7 wherein the particle sizes of the encapsulated semiconductor nanoparticles are monodispersed with deviations of less than 10% rms.

9. The functional fluorescent substance according to any of claims 1 to 8 wherein the encapsulated semiconductor nanoparticles emit a fluorescence emission spectrum of 60 nm or less in terms of the full width at half maximum (FWHM).

10. A method for staining cells or organism tissues wherein the functional fluorescent substance according to any one of claims 1 to 9 is used as a stain for cells and organism tissues.

11. An immunofluorescence method, wherein an antibody, which is labeled with the functional fluorescent substance according to any one of claims 1 to 8, is used to detect an antigen.

12. An immunofluorescence method, wherein an antigen, which is labeled with the functional fluorescent substance according to any one of claims 1 to 9, is used to detect an antibody.

13. A bioassay method, wherein the functional fluorescent substance according to any one of claims 1 to 9 is used for labeling in flow cytometry.

14. The bioassay method according to claim 13, wherein the functional fluorescent substance is imparted with identification determined by the different quantitative ratio of fluorescent dyes and a substance associated with organisms are allowed to react to detect the functional fluorescent substance by flow cytometry after the reaction.

15. A bioassay device, wherein the functional fluorescent substance according to any one of claims 1 to 9 is used for labeling in flow cytometry.
